# EUROPEAN PATENT APPLICATION

(11) **EP 3 714 920 A1**
(43) Date of publication of application: **30.09.2020**
(21) Application number: 19209479.5
(22) Date of filing: 15.11.2019
(51) Int. Cl.: A61M 5/14, F16L 3/10, F16L 3/12, A61M 39/08, G09F 3/00

(54) **PIPELINE COLLECTOR**

(30) Priority: 27.03.2019 TW 108109801
(71) Applicant: Chi Mei Medical Center, Tainan City 71004 (TW)
(72) Inventor: KUO, CHING-LUNG, 71004 Tainan City (TW); WANG, JHI-JOUNG, 71004 Tainan City (TW); TUNG, YU-CHEN, 71004 Tainan City (TW); HUANG, PEI-CHEN, 71004 Tainan City (TW); HSU, CHIA-CHEN, 71004 Tainan City (TW); HU, SHU-HUI, 71004 Tainan City (TW); WENG, JUI-YU, 710 Tainan City (TW)
(74) Representative: 2K Patentanwälte Blasberg Kewitz & Reichel

(57) **Abstract**

A pipeline collector (100) is used for bundling at least one hose (900). The pipeline collector (100) comprises at least one bundling member (10) corresponding to the hoses (900) and is characterized in that each of the bundling members (10) comprises a bundling body (11), a first top edge (12) positioned at one side of the bundling body (11), a second top edge (13) defined at the other side of the bundling body (11) relative to the first top edge (12), and a first accommodation area (14) which is recessed from the surface of the bundling body (11) relative to the hoses (900) and penetrates through the first top edge (12) and the second top edge (13), wherein a profile of the first accommodation area (14) is non-orthogonally disposed relative to the first top edge (12) or the second top edge (13).

## Description

### FIELD OF THE INVENTION

The present invention relates to a pipeline collector, in particular to a pipeline collector for sorting and collecting medical hoses in use.

### BACKGROUND OF THE INVENTION

In a hospital, inpatients, especially inpatients in an intensive care unit, are often implanted with many pipelines by medical personnel in the need for medical conditions. For example, a single patient may need a supplemental infusion tube or a drug delivery tube set in the vein; a blood collection tube and a physiological information monitoring hose set in the artery; and moreover, an implantable artificial blood vessel injection base (port-A), subcutaneous injection, and the like, and therefore the complexity is evident. If these pipelines are not properly sorted, nursing staff need to find the source along the route of a pipeline and confirm the function of the pipeline before an operation can be performed, in the course of administrating a drug or drawing blood each time. Therefore, if the medical staff can clearly see and quickly find the correct pipeline at a glance, the time of administration or infusion can be shortened, and the misjudgment of the pipeline under emergency conditions can be further reduced. In addition, a bedridden patient often needs to be turned over by the nursing staff to avoid hemorrhoids. In the course of turning over, the medical staff should carefully hold all the pipelines to avoid the pain caused by pulling, which is very inconvenient. The existing pipeline sorting device is connected in a horizontal wiring manner, and a self-adhesive label is adhered to the end of the pipeline for identification. However, the sorting device is arranged in a horizontal direction, which is not intuitive when it is recognized. In addition, the portion to which the label is adhered is rather narrow, such that the label is prone to peeling off. Therefore, there is a need for improvements in the prior art.

### SUMMARY OF THE INVENTION

An objective of the present invention is to solve the problem in the prior art that a medical hose cannot be properly sorted and easily used by nursing staff.

To fulfill said objective, the present invention provides a pipeline collector used for bundling at least one hose; the pipeline collector comprises at least one bundling member corresponding to the hoses and is characterized in that each of the bundling members comprises a bundling body, a first top edge positioned at one side of the bundling body, a second top edge defined at the other side of the bundling body relative to the first top edge, and a first accommodation area which is recessed from the surface of the bundling body relative to the hoses and penetrates through the first top edge and the second top edge, wherein a profile of the first accommodation area is non-orthogonally disposed relative to the first top edge or the second top edge.

Further, the pipeline collector further comprises a cover member detachably connected to the bundling member, the cover member comprising a cover body, and a connecting portion connected to the cover body as well as the bundling body.

Further, the cover member comprises a second accommodation area that is recessed from the cover body relative to the first accommodation area.

Further, each of the bundling members comprises a protruding portion disposed at one side of the bundling body, and an embedding portion disposed at the other side of the bundling body relative to the protruding portion and corresponds to the protruding portion.

Further, the first accommodation area is provided with an opening on the surface of the bundling body, and the opening comprises a caliber smaller than a diameter of the hoses.

Further, the protruding portion and the embedding portion are respectively disposed on any of two sides except two surfaces that the first top edge and the second top edge respectively disposed thereon, and the protruding portion of one of the bundling members is connected to the embedding portion of another one of the bundling members.

Further, the bundling body is made of a material selected from the group consisting of polypropylene, polylactic acid, and ABS resin.

Further, the pipeline collector further comprises an identification portion corresponding to the bundling members.

With the above structure, the present invention is used by the medical staff to bundle and sort pipelines while using the hoses such as an infusion tube and a blood collection tube. The medical staff can label the bundling members to clearly indicate the connection position and use of each of the hoses. Therefore, pipeline disorders can be avoided, and the time in identifying pipelines during nursing is saved. If all pipelines need to be moved at one time, the risk of inadvertent pulling or slipping can be further avoided.

### BRIEF DESCRIPTION OF THE DRAWINGS

Figure 1 is an exploded perspective view of a first embodiment in the present invention.
Figure 2 is a combined perspective view of the first embodiment in the present invention.
Figure 3 is a schematic diagram of a usage state of the first embodiment in the present invention.
Figure 4 is a schematic diagram of a usage state in which a plurality of pipeline collectors is connected in parallel in the present invention.
Figure 5 is a sectional view of Figure 4 in the present invention.
Figure 6 is a combined perspective view of a second embodiment in the present invention.
Figure 7 is a schematic diagram of a usage state of the second embodiment in the present invention.

### DETAILED DESCRIPTION OF THE PREFERRED EMBODIMENTS

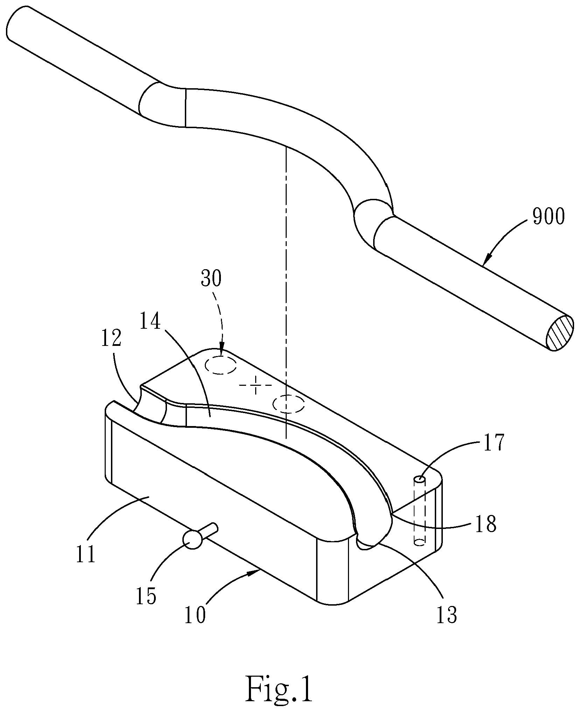

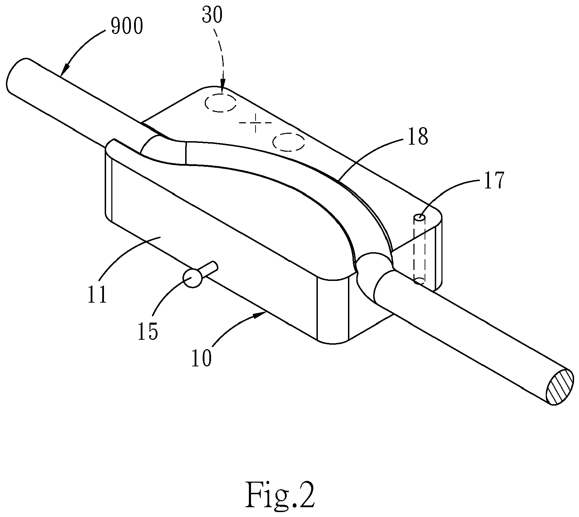

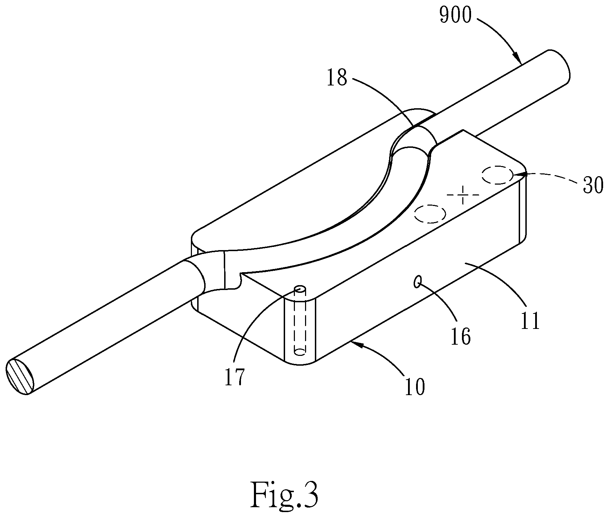

Regarding the technique of the present invention, please refer to Figures 1, 2 and 3. The present invention provides a pipeline collector 100 used for bundling at least one hose 900. In a general circumstance for using the present invention, the pipeline collector 100 should be matched with the hoses 900 which may be medical hoses, such as an infusion hose, a blood collection hose, and a hypodermic injection hose. The purpose of the hoses 900 are not the core content of the present invention and are therefore not limited herein.

Specifically, the pipeline collector 100 comprises at least one bundling member 10 corresponding to the hoses 900. Each of the bundling members 10 comprises a bundling body 11, a first top edge 12 positioned at one side of the bundling body 11, a second top edge 13 defined at the other side of the bundling body 11 relative to the first top edge 12, and a first accommodation area 14 which is recessed from the surface of the bundling body 11 relative to the hoses 900 and penetrates through the first top edge 12 as well as the second top edge 13. In this embodiment, each of the bundling members 10 is preferably made of a plastic material, such as polypropylene, polylactic acid, ABS resin, etc., to adapt to the size and shape of each of the hoses 900 for a tighter combination. In the drawings of the present invention, the bundling body 11 is in a shape of a cube as an illustrative example. The first top edge 12 and the second top edge 13 are respectively located at the top ends on both sides of the bundling body 11 and allow the first accommodation area 14 to penetrate therethrough. The first accommodation area 14 is provided with an opening 18 on the surface of the bundling body 11, and the opening 18 comprises a caliber which is smaller than the diameter of the hoses 900, so that the hoses 900 is embedded into the first accommodation area 14 and not easily dropped. Preferably, the first accommodation area 14 comprises a curvature of 1.3πrad∼1.7πrad relative to the first top edge 12 and the second top edge 13, such that the hoses 900 are constrained after being disposed in the first accommodation area 14, and not prone to peeling off. Each of the bundling members 10 is preferably manufactured by 3D printing to facilitate setting the curvature of the first accommodation area 14 and to establish an appropriate positional relationship according to the hoses 900 that is intended to be used. However, a profile of the first accommodation area 14 is non-orthogonally disposed relative to the first top edge 12 or the second top edge 13, so that a certain degree of anti-slip effect can be achieved. In addition, the pipeline collector 100 further comprises an identification portion 30 corresponding to each of the bundling members 10. The identification portion 30 may be a self-adhesive label, or a region for writing is disposed on each of the bundling members 10, so that the medical staff can quickly find a target pipeline. In addition, when each of the bundling members 10 is used, the bundling body 11 may be set in different colors to improve the recognition of each of the hoses 900 when the medical staffs are in operation. Further, the bundling body 11 comprises a through hole 17 penetrating through the bundling body 11, and a ring clamp (not shown) may be disposed in the through hole 17 for clamping to a patient.

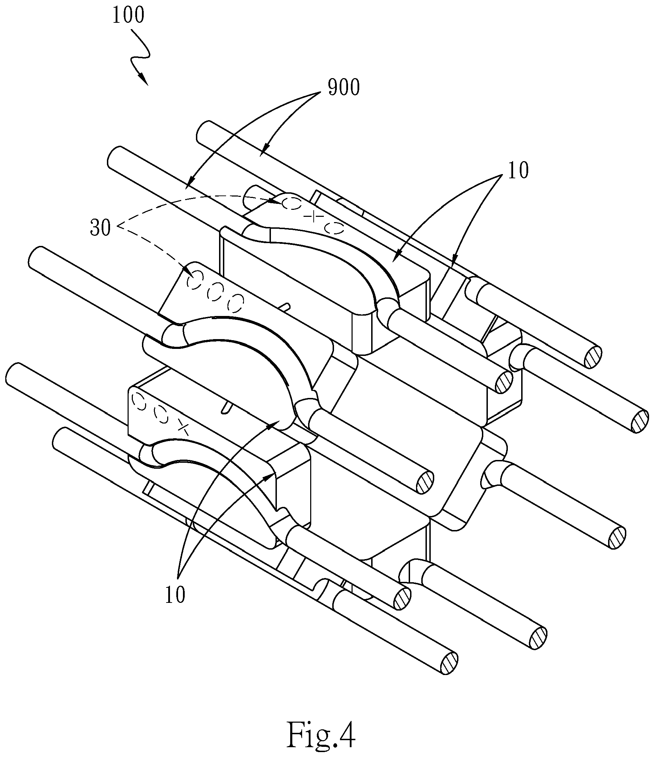

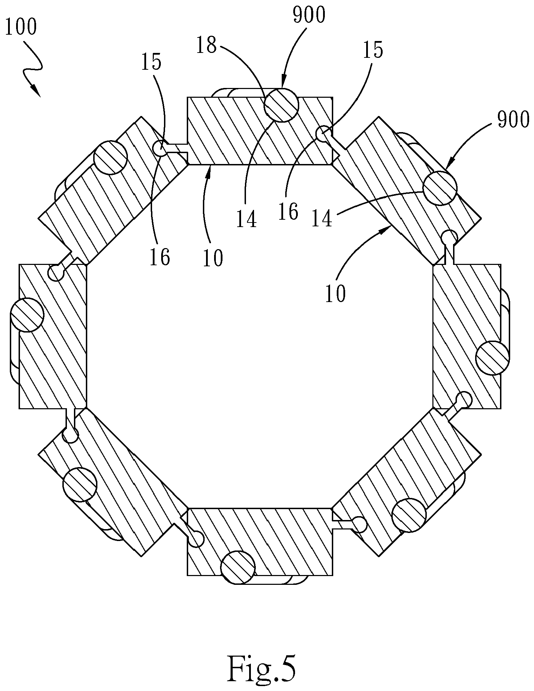

Moreover, each of the bundling members 10 comprises a protruding portion 15 disposed at one side of the bundling body 11, and an embedding portion 16 disposed at the other side of the bundling body 11 relative to the protruding portion 15. As shown in Figures 4 and 5, in this embodiment, the protruding portion 15 and the embedding portion 16 are respectively disposed on any two surfaces of the bundling body 11 except two surfaces that the first top edge 12 and the second top edge 13 respectively disposed thereon. As shown in Figure 5, the protruding portion 15of one of the bundling members 10 may connect to the embedding portion 16 of another one of the bundling members 10, so as to connect the bundling members 10 in series. Each of the protruding portions 15 and each of the embedding portions 16 may be set in different angles. Further, the bundling members 10 may be wound into an annular polygon after being connected in series, which is more convenient for the nursing staff to gather and sort.

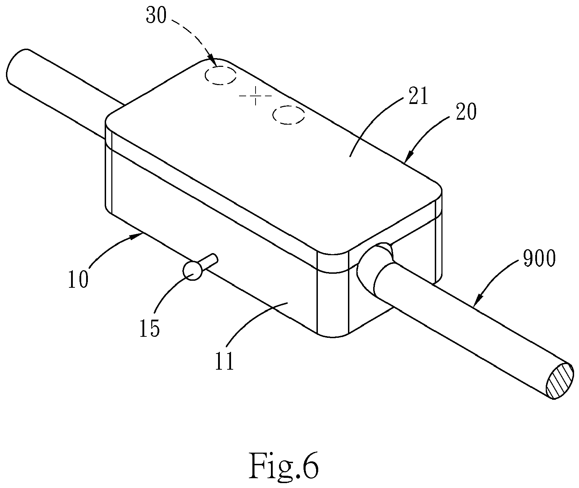

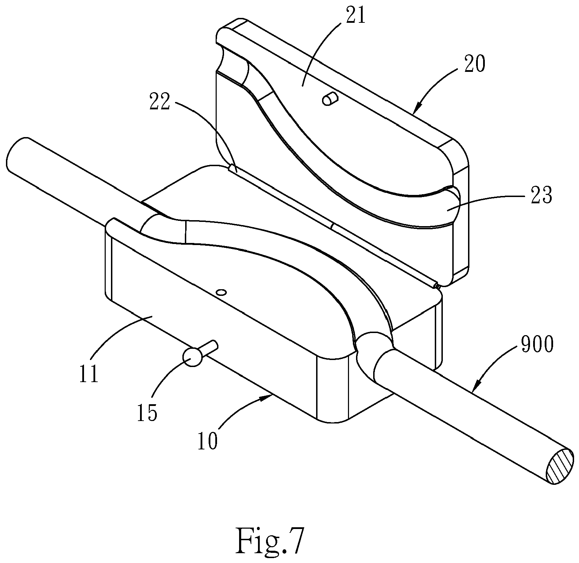

Moreover, as shown in Figures 4, 5, 6 and 7, in order to prevent the hoses 900 from falling out of the first accommodation area 14, the pipeline collector 100 further comprises a cover member 20 detachably connected to the bundling member 10. The cover member 20 comprises a cover body 21 and a connecting portion 22 connected to the cover body 21 as well as the bundling body 11. In this embodiment, the connecting portion 22 is a pivot disposed on each of the bundling members 10, so that the user can open the cover body 21 to place the hoses 900 into the first accommodation area 14, and then cover the cover body 21. In addition, in order to achieve a more reliable fixing effect, the cover member 20 comprises a second accommodation area 23 which is recessed from the cover body 21 relative to the first accommodation area 14. A combined space formed by the first accommodation area 14 and the second accommodation area 23 can completely cover the hoses 900.

With the above structure, the present invention is used by the medical staff to bundle and sort pipelines while using the hoses 900 such as an infusion tube and a blood collection tube. The medical staff can label the bundling member 10 to clearly indicate the use of each of the hoses 900. Therefore, pipeline disorders can be avoided, and the time in identifying pipelines during nursing is saved. If all pipelines need to be moved at one time, the risk of inadvertent pulling or slipping can be further avoided.

## Claims

1. A pipeline collector (100) used for bundling at least one hose (900), the pipeline collector (100) comprising at least one bundling member (10) corresponding to the at least one hose (900), wherein each of the bundling members (10) comprises:
a bundling body (11), a first top edge (12) positioned at one side of the bundling body (11), a second top edge (13) defined at the other side of the bundling body (11) relative to the first top edge (12), and a first accommodation area (14) which is recessed from the surface of the bundling body (11) relative to the hoses (900) and penetrates through the first top edge (12) and the second top edge (13), wherein a profile of the first accommodation area (14) is non-orthogonally disposed relative to the first top edge (12) or the second top edge (13).

2. The pipeline collector according to claim 1, further comprising a cover member (20) detachably connected to the bundling member (10), the cover member (20) comprising a cover body (21), and a connecting portion (22) connected to the cover body (21) as well as the bundling body (11).

3. The pipeline collector according to claim 2, wherein the cover member (20) comprises a second accommodation area (23) that is recessed from the cover body (21) relative to the first accommodation area (14).

4. The pipeline collector according to any of the preceding claims, wherein the bundling member (10) comprises a protruding portion (15) disposed at one side of the bundling body (11), and an embedding portion (16) disposed at the other side of the bundling body (11) relative to the protruding portion (15) and corresponds to the protruding portion (15).

5. The pipeline collector according to claim 4, wherein the protruding portion (15) and the embedding portion (16) are respectively disposed on any two sides of the bundling body (11) except two surfaces that the first top edge (12) and the second top edge (13) respectively disposed thereon, so that the protruding portion (15) of one of the bundling members (10) is connected to the embedding portion (16) of another one of the bundling members (10).

6. The pipeline collector according to any of the preceding claims, wherein the first accommodation area (14) is provided with an opening (18) on the surface of the bundling body (11), and the opening (18) comprises a caliber smaller than a diameter of the hoses (900).

7. The pipeline collector according to any of the preceding claims, wherein the bundling body (11) is made of a material selected from a group consisting of polypropylene, polylactic acid, and ABS resin.

8. The pipeline collector according to any of the preceding claims, further comprising an identification portion (30) corresponding to the bundling members (10).
